# FASCICULE DE BREVET EUROPEEN

(11) **EP 1 283 704 B1**
(45) Date de publication et mention de la délivrance du brevet: **18.12.2013**
(21) Numéro de dépôt: 01934105.6
(22) Date de dépôt: 11.05.2001
(51) Int. Cl.: A61K 9/00, A61K 31/192

(54) **COMPOSITIONS PHARMACEUTIQUES POUR ADMINISTRATION TRANSDERMIQUE D' AGENTS ANTI-INFLAMMATOIRES**
PHARMAZEUTISCHE ZUSAMMENSETZUNGEN ZUR TRANSDERMALEN VERABREICHUNG VON ENTZÜNDUNGSHEMMERN
PHARMACEUTICAL COMPOSITIONS FOR TRANSDERMAL ADMINISTRATION OF ANTI-INFLAMMATORY AGENTS

(30) Priorité: 12.05.2000 FR 0006048
(43) Date de publication de la demande: 19.02.2003
(73) Titulaire: SANOFI, 75008 Paris (FR)
(72) Inventeur: ILLEL, Brigitte, F-34070 Montpellier (FR); VERGNAUD, Jean-Pierre, F-34070 Montpellier (FR)
(74) Mandataire: Thouret-Lemaitre, Elisabeth
(86) Numéro de dépôt international: PCT/FR2001/001442
(87) Numéro de publication internationale: WO 2001/085143

(56) Documents cités:
- EP-A- 0 450 123
- WO-A-96/30000
- WO-A-99/39713
- WO-A1-99/09954
- US-A- 5 350 769
- DATABASE CHEMABS [en ligne] CHEMICAL ABSTRACTS SERVICE, COLUMBUS, OHIO, US; SAITO, HIROSHI ET AL: "Topical gel preparations containing absorption accelerators, and" retrieved from STN Database accession no. 117:239871 HCA XP002157095 & JP 04 234314 A (MIKASA SEIYAKU K. K., JAPAN) 24 août 1992 (1992-08-24)
- DATABASE CHEMABS [en ligne] CHEMICAL ABSTRACTS SERVICE, COLUMBUS, OHIO, US; TERUMO CORP., JAPAN: "Film -forming topical formulations" retrieved from STN Database accession no. 102:137818 HCA XP002157096 & JP 59 216822 A (TERUMO CORP., JAPAN) 6 décembre 1984 (1984-12-06)
- SANTUS G C ET AL: "TRANSDERMAL ENHANCER PATENT LITERATURE" JOURNAL OF CONTROLLED RELEASE,NL,ELSEVIER SCIENCE PUBLISHERS B.V. AMSTERDAM, vol. 25, no. 1 / 02, 27 mai 1993 (1993-05-27), pages 1-20, XP000361364 ISSN: 0168-3659

## Description

La présente invention se rapporte, d'une manière générale, à une nouvelle composition pharmaceutique pour administration transdermique.

Plus précisément, l'invention concerne une composition pharmaceutique pour administration transdermique d'ibuprofène, composition capable de former un film souple après séchage sur la peau.

L'administration transdermique de principes actifs médicamenteux représente une technique séduisante, car non invasive, douée d'avantages certains tels qu'absence d'effets secondaires gastro-intestinaux ou d'altérations de la substance active par les enzymes hépatiques.

Pour être efficace, cette technique doit toutefois permettre une pénétration transcutanée du médicament sur une période prolongée et de manière suffisante pour atteindre des taux plasmatiques compatibles avec un traitement thérapeutique.

A ce jour, divers systèmes ou dispositifs pour ce type d'administration ont été proposés qui permettent d'introduire, dans le flux sanguin, des doses contrôlées de substances médicamenteuses.

On connaît par exemple le dispositif d'administration transdermique appelé communément "patch" constitué d'un réservoir formé de matériaux plastiques synthétiques contenant le principe actif. Ce réservoir peut être recouvert, sur sa face en contact avec la peau, par une membrane microporeuse dont la perméabilité à la substance active régule sa diffusion et par conséquent son dosage.

En dépit des réelles possibilités offertes par ce dispositif on peut lui préférer d'autres systèmes. En effet il est connu que le patch peut se décoller de la peau et, par ailleurs, présenter un aspect souvent inesthétique.

Des gels contenant différents principes actifs ont également été proposés. Toutefois, cette forme pharmaceutique peut présenter certains inconvénients à l'usage, généralement un toucher collant désagréable pour le patient de même qu'une maîtrise difficile de la dose de principe actif administrée et un contrôle délicat de la surface de recouvrement.

D'autres systèmes ont également été rapportés qui aident à l'administration transdermique de principes médicamenteux.

A ce titre, on peut citer des compositions pulvérisables comportant notamment des polymères capables de former un film au contact de la peau et de relarguer le principe actif pour une administration transcutanée. Des compositions de ce type, décrites par exemple dans le brevet EP 0319555, comprennent un principe actif, une matrice polymérique formant un film souple après séchage, un solvant contrôlant le relargage de la substance active à savoir un sorbitane macrogollaurate, une paraffine, un diglycéride ou triglycéride d'acide gras à chaîne moyenne ou le carbonate de propylène ainsi qu'un solvant, pour la matrice, capable de s'évaporer sur la peau et enfin un agent propulseur permettant de pulvériser cette composition renfermée dans un dispositif approprié.

Une matrice constituée d'éthylcellulose y est cependant déconseillée en raison de sa tendance à obturer le système de pulvérisation.

En outre, des compositions telles que proposées par le susdit brevet, caractérisées par la présence d'un gaz propulseur par exemple un hydrocarbure halogéné, sont de plus en plus controversées suite aux risques potentiels qu'elles sont susceptibles de provoquer pour l'environnement.

Au surplus, les compositions du brevet EP 0319555, par la présence de dérivés polyméthacryliques, dégagent une odeur caractéristique assez désagréable pour la patient ou son entourage.

D'autres compositions pharmaceutiques pour administration topique contenant un principe actif, un solvant et divers autres ingrédients sont également connues.

A titre d'exemple, on peut citer le brevet EP 55396 qui décrit des compositions antimycotiques formées :
- d'un éther de cellulose
- de 2 à 10% d'un agent d'étalement tel que le myristate d'isopropyle ou le palmitate d'isopropyle
- de 1 à 8% d'un agent de solubilisation
- de 0,05 à 1 % d'un principe actif
- et d'un solvant tel que l'isopropanol

Toutefois, ces compositions si elles peuvent être utilisées pour des applications topiques dermatologiques se révèlent totalement inadaptées pour une application par pulvérisation même après ajout de 10 à 40% d'un gaz propulseur comme recommandé, car elles apparaissent trop visqueuses et susceptibles d'amener divers inconvénients tels que bouchage du dispositif de pulvérisation.

On peut également citer le brevet EP 319964 décrivant des compositions antifongiques capables de former un film comprenant :
- de 0,1 à 1,5% de tolnaphtalate
- de 10 à 20% d'un copolymère méthacrylate de diméthylaminoéthyle / méthacrylate
- de 0,5 à 10% d'un ester d'acide gras
- un solvant de type alcool et éventuellement de 0,1 à 5% d'un dérivé cellulosique.

Cette composition ne semble pas non plus adaptée pour une pulvérisation. En outre, comme déjà évoqué précédemment, la présence de dérivés méthacryliques lui procure une odeur rédhibitoire.

D'autre part, on peut signaler le brevet EP 289900 qui se rapporte à des compositions antibactériennes à usage topique comprenant :
- de 0,5 à 10% d'un principe actif antibactérien
- de 1 à 30% d'un polymère non hydrosoluble notamment l'éthylcellulose ou un copolymère de polyvinylpyrrolidone
- de 0,5 à 40% d'un plastifiant, généralement une huile essentielle, jouant également le rôle de promoteur d'absorption transcutanée
- de 50 à 95% d'un solvant tel que l'éthanol

Comme on le sait, les huiles essentielles sont constituées en grande majorité de dérivés terpéniques.

Dans le cadre de l'invention, on a expérimenté, une composition analogue à celle décrite dans ce brevet, contenant notamment l'estradiol comme principe actif et le limonène, qui est un terpène, comme promoteur d'absorption transdermique. Toutefois, une telle composition n'a fourni que de très faibles flux de diffusion transcutanée de ce principe actif.

Au surplus, on peut signaler la demande de brevet EP 0581587 qui décrit une composition pharmaceutique concernant de l'estradiol, de l'hydroxypropylcellulose, du myristate d'isopropyle, de l'eau et de l'éthanol.

Toutefois, cette composition qui contient 15% en poids d'hydroxypropylcellulose ne pourrait être pulvérisée en raison de la quantité trop importante de ce dérivé cellulosique. Cette composition se présente d'ailleurs sous forme de gel. La demande de brevet WO99/09954 décrit des compositions topiques comprenant de l'ibuprofène, sous forme de gels hydroalcooliques.

Enfin, on peut mentionner la demande de brevet WO 96/30000 qui décrit des compositions filmogènes pour administration transdermique comprenant :
- jusqu'à 6% d'une matrice formée de polymères ou copolymères cellulosiques
- de 0,1 à 20% en poids d'un principe actif
- de 15 à 30% en poids d'un promoteur d'absorption transcutanée choisi parmi des acides gras aliphatiques ou des alcools gras aliphatiques
- de 44 à 84,9% d'un solvant non aqueux

Des exemples ont été décrits dans cette demande de brevet qui illustrent des compositions contenant l'éthylcellulose comme matrice, l'ibuprofène comme principe actif, le 2-éthyl-hexanoate de 2-éthyl-hexyle comme promoteur d'absorption transcutanée et l'éthanol comme solvant non aqueux.

Toutefois ces compositions présentent une stabilité précaire dans le temps qui les rend difficilement utilisables quelques années après leur fabrication notamment après trois années.

La recherche d'une composition permettant la diffusion transdermique de principes actifs médicamenteux, notamment l'ibuprofène, à des taux compatibles avec un traitement thérapeutique tout en étant exempte des inconvénients rapportés précédemment reste d'un intérêt majeur.

Dans le cadre de la présente invention, on a effectué des essais préliminaires visant essentiellement à étudier la solubilité de l'ibuprofène dans différents solvants ou composants. On a pu ainsi vérifier que ce principe actif est soluble dans des mélanges esters d'acide gras/alcools tels que ceux décrits dans la demande de brevet WO 96/30000 notamment des mélanges 2-éthyl-hexanoate de 2-éthyl-hexyle/éthanol ou isopropanol, de manière à former des solutions homogènes.

Toutefois, l'ibuprofène s'est révélé totalement insoluble dans l'eau.

Or, il a été découvert de manière surprenante, que l'ajout d'eau aux mélanges acides gras/alcool de la demande de brevet précitée, en particulier l'ajout d'eau aux mélanges 2-éthyl-hexanoate de 2-éthyl-hexyle/isopropanol, permet d'augmenter la solubilité de l'ibuprofène dans ces mélanges esters d'acides gras/alcool et de fournir des compositions pharmaceutiques particulièrement stables et dépourvues des inconvénients cités ci-dessus, tout en étant capables de délivrer ce principe actif dans le flux sanguin à des taux atteignant largement des seuils thérapeutiques.

Ainsi, l'invention a pour objet principal une composition pharmaceutique pour administration transdermique comprenant :
- une matrice polymérique de relargage d'éthylcellulose capable de former un film souple après séchage,, cette matrice étant présente à raison de 0.5% à 2% du poids de la composition
- **de l'ibuprofène** à titre de principe actif
- un promoteur d'absorption transcutanée du principe actif, choisi parmi :
   (i) un ester d'acide gras aliphatique, soluble dans le ou les solvants non aqueux physiologiquement acceptables, ayant au total de 10 à 30 atomes de carbone et étant éventuellement substitué par un ou deux groupements hydroxyles, carboxyliques ou acyloxy en C₁ - C₄ ou éventuellement interrompu par une ou deux liaisons éthyléniques ou par une ou deux oxygènes éther,
   (ii) un alcool gras aliphatique en C₁₀ - C₃₀, soluble dans le ou les solvants non aqueux physiologiquement acceptables, et éventuellemnt substitué par un ou deux groupements hydroxyles, carboxyliques ou acyloxy en C₁ - C₄ ou étant éventuellement interrompu par une ou deux liaisons éthyléniques ou par un ou deux oxygènes éther
- de l'eau, présente à raison de 3% à 10% du poids de la composition
- **au moins l'isopropanol** à titre de solvant non aqueux physiologiquement acceptable capable de dissoudre la matrice de relargage, le principe actif et le promoteur d'absorption transcutanée ainsi que de s'éliminer rapidement par évaporation au contact de la peau.

La matrice polymérique est l'éthylcellulose, capable à la fois de former un film souple après évaporation du solvant et de relarguer le principe actif.

En outre, cette matrice est présente à une concentration de 0,5% à 2% du poids de la composition selon l'invention. Préférentiellement, on utilise de 0,5% à 1% en poids de matrice notamment 0,5%.

L'éthylcellulose présente aprés séchage une résistance à l'abrasion et une stabilité mécanique appropriées. Pour cette raison, des matrices cellulosiques de ce type pourront être rincées à l'eau sans crainte de détérioration.

L'éthylcellulose est la matrice polymérique de relargage de choix pour la formation d'un film souple au contact de la peau.

Le principe actif est l'ibuprofène.

L'ibuprofène est incorporé dans les compositions de l'invention à des concentrations ne dépassant pas 15% du poids de ces compositions notamment de 1% à 15%, préférentiellement de 3% à 10% étant entendu que le principe actif sera introduit à des concentrations individualisées et connues de l'état de la technique pour une administration transdermique ou adaptées à cette voie d'adminstration.

Par exemple, l'ibuprofène peut figurer dans les compositions de l'invention à raison de 4% à 10% du poids de cette composition notamment de 4% à 5%, de préférence 5%.

De manière à atteindre une diffusion transcutanée suffisante pour obtenir l'efficacité thérapeutique du principe actif, on associe à la matrice polymérique et à ce principe actif, un promoteur d'absorption transcutanée dont la concentration ne dépassera pas 40% en poids de la composition. Ce dernier entre dans les compositions de l'invention avantageusement à raison de 10% à 30% du poids de cette composition, de préférence de 15% à 25% par exemple 20%.

Le promoteur d'absorption transcutanée en question, pour être efficace, doit être capable de désorganiser de façon transitoire la barrière cutanée de manière à augmenter la perméabilité de la peau sans l'irriter tout en favorisant la diffusion du principe actif choisi selon une cinétique et une concentration suffisante pouvant être maintenue pendant un certain temps.

Ce promoteur d'absorption transcutanée sera sélectionné parmi des substances solubles dans les solvants non aqueux physiologiquement acceptables, capables de s'évaporer rapidement au contact de la peau.

On le choisira parmi les composés suivants qui présentent le degré de solubilité nécessaire dans le ou les solvants non aqueux en question et qui réunissent les meilleures qualités rapportées ci-dessus c'est-à-dire parmi :
- des esters d'acide gras aliphatiques, essentiellement des esters ayant au total de 10 à 30 atomes de carbone éventuellement substitués par un ou deux groupements hydroxyles, carboxyliques ou acyloxy en C₁ - C₄ tel qu'acétoxy ou éventuellement interrompus par une ou deux liaisons éthyléniques ou par un ou deux oxygènes éther,
- des alcools gras aliphatiques, essentiellement des alcools en C₁₀ - C₃₀ éventuellement substitués par un ou deux groupements hydroxyles, carboxyliques ou acyloxy en C₁ - C₄ tel qu'acétoxy ou éventuellement interrompus par une ou deux liaisons éthyléniques ou par un ou deux oxygènes éther.

Des promoteurs d'absorption particulièrement préférés qui peuvent être sélectionnés parmi les esters d'acides gras aliphatiques et alcools gras aliphatiques mentionnés précédemment sont rapportés ci-dessous, à savoir :
a) des esters d'acides gras aliphatiques de formule générale : 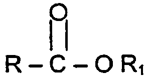 dans laquelle R représente un groupement alkyle ou alcényle, linéaire ou ramifié, en C₂ - C₁₇ éventuellement substitué par un groupement hydroxyle, carboxylique ou acyloxy en C₁ - C₄ et R₁ représente un groupement alkyle, linéaire ou ramifié, en C₃ - C₈ éventuellement substitué par un ou deux groupements hydroxyles tel que par exemple un groupement isopropyle, 2-éthyl-hexyle, 1,2-dihydroxy-éthyle ou R₁ représente un groupement -CH₂-CH₂-O-(CH₂)₂-O-CH₂-CH₃, l'ester d'acide gras aliphatique comportant un minimum de 10 atomes de carbone et un maximum de 2 groupements hydroxyles,
b) des alcools gras aliphatiques de formule générale :

   R₂-OH

   dans laquelle R₂ représente un groupement alkyle en C₁₀-C₂₀.

A titre de composés particuliers ayant montré les meilleures potentialités pour promouvoir l'absorption transcutanée de principes actifs, notamment l'ibuprofène, on peut citer :
le 2-éthyl-hexanoate de 2-éthyl-hexyle
le myristate d'isopropyle
le myristate de l'éther monoéthylique du diéthylèneglycol
le palmitate d'isopropyle
le 2-octyldodécanol
l'undécylénate de 2-éthyl-hexyle
le succinate de 2-éthyl-hexyle
le 12-hydroxy-stéarate de 2-éthyl-hexyle
le 12-acétoxy-stéarate de 2-éthyl-hexyle
l'isostéarate de glycérol
le laurate d'hexyle

Le 2-éthyl-hexanoate de 2-éthyl-hexyle représente le promoteur d'absorption préféré notamment pour des compositions transdermiques selon l'invention dont le principe actif est l'ibuprofène.

L'eau, quant à elle, est introduite dans les compositions de l'invention à une concentration compatible avec la quantité de polymères ou de copolymères cellulosiques choisis et avec un temps de séchage acceptable de la composition pulvérisée sur la peau.

Cette concentration en eau sera de 3% à 10% en poids notamment de 3% à 5%.

En ce qui concerne le ou les solvants non aqueux physiologiquement acceptables, capables de dissoudre la matrice de relargage, le principe actif et le promoteur d'absorption transcutané, on le ou les choisira parmi des composés de point d'ébullition relativement faible, à savoir inférieur à 100°C à pression atmosphérique, de façon qu'ils puissent s'éliminer rapidement par évaporation au contact de la peau et aider de la même manière à la formation d'un film par séchage sans toutefois provoquer d'irritation locale.

De tels solvants physiologiquement acceptables sont généralement utilisés en quantité suffisante pour atteindre 100% du poids de la composition finale habituellement à des concentrations supérieures à 50% du poids de cette composition. En outre, ils peuvent être sélectionnés parmi des composés volatils tels que le dichlorométhane, l'éthanol, l'isopropanol ou l'acétate d'éthyle.

L'isopropanol représente le solvant préféré selon l'invention.

En conséquence, selon un de ses aspects particuliers, l'invention concerne une composition transdermique comprenant en poids :
- de 0,5% à 2% d'éthylcellulose
- de 3% à 10% d'ibuprofène, en particulier de 3% à 6% d'ibuprofène
- de 10% à 30% d'un promoteur d'absorption transcutanée du principe actif, en particulier de 15% à 25% d'un ester d'acide gras ou d'un alcool gras choisi parmi :
   le 2-éthyl-hexanoate de 2-éthyl-hexyle
   le myristate d'isopropyle
   le myristate de l'éther monoéthylique du diéthylèneglycol
   le palmitate d'isopropyle
   le 2-octyldodécanol
   l'undécylénate de 2-éthyl-hexyle
   le succinate de 2-éthyl-hexyle
   le 12-hydroxystéarate de 2-éthyl-hexyle
   le 12-acétoxystéarate de 2-éthyl-hexyle
   l'isostéarate de glycerol
   le laurate d'hexyle
- de 3% à 10% d'eau
- une quantité suffisante, pour atteindre 100% du poids de la composition, d'au moins l'isopropanol à titre de solvant non aqueux physiologiquement acceptable capable de dissoudre la matrice de relargage, le principe actif et le promoteur d'absorption transcutanée ainsi que de s'éliminer rapidement par évaporation au contact de la peau.

Si nécessaire, les compositions de l'invention comprendront également une fraction aromatisante constituée d'un ou de plusieurs composés aromatisants tels que le camphre ou de préférence le lévomenthol.

Cette fraction aromatisante sera utilisée à des concentrations ne dépassant pas 5% en poids de la composition notamment pour la sensation de fraîcheur qu'elle pourrait procurer sur la peau. A titre d'exemple, le lévomenthol pourra être présent dans les compositions de l'invention à la concentration de 2% en poids de la composition.

Des caractéristiques et avantages des compositions de l'invention apparaîtront à la lumière de la description ci-après.

### Tests de solubilité

On a effectué des tests comparatifs en vue de déterminer la solubilité à saturation de l'ibuprofène dans différents composants de la formulation transdermique de l'invention en l'absence de matrice, à savoir l'eau, le 2-éthyl-hexanoate de 2-éthyl-hexyle comme promoteur d'absorption transcutanée, ci-après dénommé « promoteur » et l'isopropanol comme solvant non aqueux.

Ces différents composants ont été utilisés seuls ou en mélanges homogènes formant une solution limpide et les mesures de solubilité ont été pratiquées à 30°C, après 24 heures d'agitation magnétique et après filtration sur filtre de 0,22 µm.

On a obtenu les résultats suivants :

### a) Composants seuls

| Eau | Isopropanol | Promoteur | Concentration saturante ibuprofène |
|---|---|---|---|
| (% m/m) | (% m/m) | (% m/m) | (mg/ml) |
| 100 | - | - | 0,069 |
| - | 100 | - | 441 |
| - | - | 100 | 192 |

### b) Composants en mélanges binaires ou ternaires

| Eau | Isopropanol | Promoteur | Concentration saturante ibuprofène |
|---|---|---|---|
| (% m/m) | (% m/m) | (% m/m) | (mg/ml) |
| - | 68,21 | 31,79 | 403 |
| 4,38 | 68,16 | 27,46 | 431 |
| 8,54 | 68,23 | 23,23 | 436 |
| - | 89,31 | 10,69 | 424 |
| 4,33 | 85 | 10,67 | 451 |
| 8,59 | 80,7 | 10,69 | 448 |

Ces résultats montrent que :
- si une fraction pondérale d'isopropanol est remplacée par du promoteur, composant dans lequel l'ibuprofène est plus de deux fois moins soluble, la solubilité de ce principe actif dans ces mélanges binaires homogènes est diminuée ;
- si une fraction pondérale d'isopropanol ou de promoteur dans leurs mélanges binaires homogènes est remplacée par de l'eau, composant dans lequel l'ibuprofène est quasi insoluble, la solubilité de ce principe actif dans ces mélanges ternaires homogènes est, au contraire, augmentée.

L'ensemble des substances entrant dans les compositions de l'invention pour administration transdermique constituent des produits connus ou pouvant être préparés par des méthodes connues, certains de ces produits étant disponibles dans le commerce.

Ces compositions selon l'invention peuvent être préparées, de manière classique, en mélangeant les divers constituants dans des proportions choisies de manière à former des mélanges homogènes.

Par exemple, on peut, sous agitation, dissoudre le promoteur d'absorption transcutanée dans le ou les solvants physiologiquement acceptables contenant l'eau puis ajouter le principe actif et finalement la matrice de relargage.

Les compositions transdermiques de l'invention ainsi obtenues peuvent être appliquées par tout moyen sur une zone cutanée prédéterminée notamment et de préférence par pulvérisation directe au moyen d'une pompe doseuse de type connu et commercialisé sans l'aide d'agent propulseur tel qu'un gaz comprimé ou liquéfié.

Comme l'ajout d'eau et de polymère ou copolymère cellulosique tel que l'éthylcellulose à un mélange de principe actif tel que l'ibuprofène, de promoteur d'absorption transcutanée tel que le 2-éthyl-hexanoate de 2-éthyl-hexyle et d'un alcool tel que l'ispropanol augmente la tension superficielle de l'ensemble, on veillera à respecter une tension superficielle de la composition finale compatible avec une pulvérisation de qualité.

Bien que l'état de la technique affirme l'inverse, on a remarqué, de manière surprenante, qu'une matrice de relargage formée d'éthylcellulose, toutefois aux concentrations préconisées dans le cadre de l'invention, ne provoque pas d'obstruction par collage à la sortie de l'embout de la tête pulvérisatrice, de sorte que les compositions de l'invention pourront être pulvérisées sans nécessité de gaz propulseur et sans crainte de détérioration du récipient pulvérisateur.

Si on le désire, on peut néanmoins administrer les compositions de l'invention par pulvérisation à partir d'un récipient muni d'une valve doseuse, contenant en surplus un gaz propulseur comprimé tel que l'azote ou le protoxyde d'azote, ou liquéfié tel que le butane.

Les compositions filmogènes de l'invention présentent des avantages incontestables puisqu'elles sont capables de provoquer la diffusion transcutanée suffisante d'ibuprofène, de manière à produire des taux thérapeutiques contrairement aux taux libérés par les compositions et matrices pour compositions transdermiques connues telles que celles décrites par exemple dans le brevet EP 0319555.

Par ailleurs, les compositions de l'invention se sont révélées de grande stabilité au contraire de compositions exemplifiées dans la demande de brevet WO96/30000. En effet, trois années après leur fabrication des compositions de l'invention contenant l'ibuprofène comme principe actif, en particulier la composition préférée ci-dessous :

| | % en poids |
|---|---|
| • Ethylcellulose | 0,5 |
| • Ibuprofène | 5 |
| • 2-Ethylhexanoate de 2-éthyl-hexyle | 20 |
| • Eau | 4 |
| • Isopropanol | 70,5 |

devraient présenter moins de 5% en poids de produits de transformation de l'ibuprofène alors que les taux des produits correspondants issus de l'ibuprofène présent dans les compositions antérieures devraient être largement supérieurs.

En outre, les compositions selon l'invention, tout en étant dépourvues de toute odeur désagréable, s'étalent en un film homogène sur la zone cutanée sélectionnée et, à cette fin, ne nécessitent pas forcément l'intermédiaire d'agents gazeux propulseur nocifs pour l'environnement.

Ces films sont suffisamment souples et résistants à l'abrasion pour éviter toute détérioration sur la peau d'un patient et présentent une meilleure tolérance que les dispositifs transdermiques connus puisqu'en raison de leur minceur et de l'absence de tout recouvrement les échanges gazeux et aqueux avec l'extérieur ne sont pas forcément perturbés.

Enfin, les compositions de l'invention, sous forme de film souple, procurent un meilleur confort pour le patient qu'un patch transdermique et, grâce à leur transparence, sont totalement invisibles.

Les Exemples, non limitatifs suivants, illustrent la préparation de compositions de l'invention.

### EXEMPLE 1

### Composition transdermique contenant l'ibuprofène

On prépare 100 g d'une composition transdermique de formulation suivante :

| | % en poids |
|---|---|
| Ethylcellulose 6mPa.sec | 0,5% |
| Ibuprofène | 5% |
| 2-Ethyl-hexanoate de 2-éthyl-hexyle | 20% |
| Isopropanol | 70,5% |
| Eau | 4% |

en mélangeant rapidement et sous agitation magnétique 70,5 g d'isopropanol, 4 g d'eau et 20 g de 2-éthyl-hexanoate de 2-éthyl-hexyle.

On ajoute alors, par petites fractions, 5 g d'ibuprofène au mélange obtenu puis, après dissolution complète (5 minutes), on introduit 0,5 g d'éthylcellulose 6mPa.sec, sous vive agitation, afin d'éviter la formation de grumeaux. La solution finale obtenue est homogène légèrement opalescente.

En vue d'une administration par pulvérisation, on remplit des boîtiers à l'aide de 50 ml de la solution précédemment obtenue et on les munit d'une vasopompe à sertir comportant un bouton poussoir.

On actionne deux fois la pompe pour l'amorcer avant sa première utilisation.

### EXEMPLES 2 à 6

### Compositions transdermiques contenant l'ibuprofène

En utilisant le même procédé que dans l'Exemple 1, on a préparé les compositions transdermiques de formulations suivantes :

| | | % en poids |
|---|---|---|
| **EX.2** | Ethylcellulose | 0,5% |
| | Ibuprofène | 5% |
| | Myristate d'isopropyle | 20% |
| | Isopropanol | 70,5% |
| | Eau | 4% |
| **EX. 3** | Ethylcellulose | 2% |
| | Ibuprofène | 5% |
| | 2-Ethyl-hexanoate de 2-éthyl-hexyle | 20% |
| | Isopropanol | 69% |
| | Eau | 4% |
| **EX. 4** | Ethylcellulose | 0,5% |
| | Ibuprofène | 10% |
| | 2-Ethyl-hexanoate de 2-éthyl-hexyle | 20% |
| | Isopropanol | 65,5% |
| | Eau | 4% |
| **EX. 5** | Ethylcellulose | 0,5% |
| | Ibuprofène | 5% |
| | Succinate de 2-éthyl-hexyle | 20% |
| | Isopropanol | 70,5% |
| | Eau | 4% |
| **EX. 6** | | |
| | Ethylcellulose | 0,5% |
| | Ibuprofène | 5% |
| | 2-Ethyl-hexanoate de 2-éthyl-hexyle | 20% |
| | Eau | 4% |
| | Isopropanol | 68,5% |
| | Lévomenthol | 2% |

## Revendications

1. Composition pharmaceutique pour administration transdermique, **caractérisée en ce qu'**elle comprend :
• une matrice polymérique de relargage d'éthylcellulose capable de former un film souple après séchage" cette matrice étant présente à raison de 0,5% à 2% du poids de la composition
• **de l'ibuprofène** à titre de principe actif
• un promoteur d'absorption transcutanée du principe actif, choisi parmi :
(i) un ester d'acide gras aliphatique, soluble dans le ou les solvants non aqueux physiologiquement acceptables, ayant au total de 10 à 30 atomes de carbone et étant éventuellement substitué par un ou deux groupements hydroxyles, carboxyliques ou acyloxy en C₁ - C₄ ou éventuellement interrompu par une ou deux liaisons éthyléniques ou par une ou deux oxygènes éther,
(ii) un alcool gras aliphatique en C₁₀ - C₃₀, soluble dans le ou les solvants non aqueux physiologiquement acceptables, et éventuellemnt substitué par un ou deux groupements hydroxyles, carboxyliques ou acyloxy en C₁ - C₄ ou étant éventuellement interrompu par une ou deux liaisons éthyléniques ou par un ou deux oxygènes éther
• de l'eau, présente à raison de 3% à 10% du poids de la composition
• **au moins l'Isopropanol** à titre de solvant non aqueux physiologiquement acceptable capable de dissoudre la matrice de relargage, le principe actif et le promoteur d'absorption transcutanée ainsi que de s'éliminer rapidement par évaporation au contact de la peau.

2. Composition pharmaceutique selon la Revendication 1, **caractérisée en ce que** la matrice polymérlque de relargage est présente à raison de 0,5% à 1% du poids de la composition.

3. Composition pharmaceutique selon une des Revendications 1 ou 2, **caractérisée en ce que** la principe actif est présent à une concentration ne dépassant pas 15% du poids de la composition.

4. Composition pharmaceutique selon la Revendication 3, **caractérisée en ce que** le principe actif est présent à raison de 3% à 10% du poids de la composition.

5. Composition pharmaceutique selon une des Revendications 1 à 4, **caractérisée en ce que** le promoteur d'absorption transcutanée est présent à une concentration ne dépassant pas 40% du poids de la composition.

6. Composition pharmaceutique selon la Revendication 5, **caractérisée en ce que** le promoteur d'absorption transcutanée est présent à raison de 10% à 30% du poids de la composition.

7. Composition pharmaceutique selon la Revendication 6, **caractérisée en ce que** le promoteur d'absorption transcutanée est présent à raison de 15% à 25% du poids de la composition.

8. Composition pharmaceutique selon une des Revendications 1 à 7, **caractérisée en ce que** le solvant non aqueux physiologiquement acceptable est présent en quantité suffisante pour atteindre 100% du poids de la composition.

9. Composition pharmaceutique selon l'une des Revendications 1 à 8, **caractérisée en ce que** l'ibuprofène est présent à raison de 4% à 10% du poids de la composition.

10. Composition pharmaceutique selon la Revendication 9, **caractérisée en ce que** l'ibuprofène est présent à raison de 4% à 5% du poids de la composition.

11. Composition pharmaceutique selon la Revendication 10, **caractérisée en ce que** le promoteur d'absorption transcutanée est choisi parmi :
• un ester d'acide gras aliphatique soluble dans le ou les solvants non aqueux physiologiquement acceptables et de formule générale : 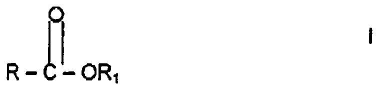 dans laquelle R représente un groupement alkyle ou alcényle, linéaire ou ramifié, en C₂ - C₁₇ éventuellement substitué par un groupement hydroxyle, carboxylique ou acyloxy en C₁ - C₄ et R₁ représente un groupement alkyle, linéaire ou ramifié, en C₃ - C₈ éventuellement substitué par un ou deux groupements hydroxyles ou R₁ représente un groupement -CH₂-CH₂-O-(CH₂)₂-O-CH₂-CH₃, l'ester d'acide gras aliphatique comportant un minimum de 10 atomes de carbone et un maximum de 2 groupements hydroxyles,
• un alcool gras aliphatique, soluble dans le ou les solvants non aqueux physiologiquement acceptables et de formule générale :
R₂-OH II
dans laquelle R₂ représente un groupement alkyle en C₁₀-C₂₀.

12. Composition pharmaceutique selon la Revendication 11, **caractérisée en ce que** R₁ représente un groupement isopropyle, 2-éthyl-hexyle ou 1, 2-dihydroxyéthyle.

13. Composition pharmaceutique selon une des Revendications 9 ou 10, **caractérisée en ce que** le promoteur d'absorption transcutanée est choisi parmi les :
• 2-éthyl-hexanoate de 2-éthyl-hexyle
• myristate d'isopropyle
• myristate de l'éther monoéthylique du diéthylèneglycol
• palmitate d'isopropyle
• 2-octyldodécanol
• undécylénate de 2-éthyl-hexyle
• succinate de 2-éthyl-hexyle
• 12-hydroxy-stéarate de 2-éthyl-hexyle
• 12-acétoxy-stéarate de 2-éthyl-hexyle
• isostéarate de glycérol
• laurate d'hexyle

14. Composition pharmaceutique selon la Revendication 10, 11 ou 12, **caractérisée en ce que** le promoteur d'absorption transcutanée est le 2-éthyl-hexanoate de 2-éthyl-hexyle.

15. Composition pharmaceutique pour administration transdermique **caractérisée en ce qu'**elle comprend, en poids :
a) de 0,5% à 2% d'une matrice polymérique de relargage d'ethylcellulose capable de former un film souple après séchage,
b) de 3% à 10% d'ibuprofène à titre de principe actif
c) de 10% à 30% d'un promoteur d'absorption transcutanée du principe actif choisi parmi les :
• 2-éthyl-hexanoate de 2-éthyl-hexyle
• myristate d'isopropyle
• myristate de l'éther monoéthylique du diéthylèneglycol
• palmitate d isopropyle
• 2-octyldodécanol
• undécylénate de 2-éthyl-hexyle
• le succinate de 2-éthyl-hexyle
• le 12-hydroxy-stéarate de 2-éthyl-hexyle
• laurate d'hexyle
• isostéarate de glycérol
• 12-acétoxy-stéarate de 2-éthyl-hexyle
d) de 3% à 10% d'eau
e) une quantité suffisante, pour atteindre 100% du poids de la composition, d'au moins l'isopropanol à titre de solvant non aqueux physiologiquement acceptable capable de dissoudre la matrice de relargage, le principe actif et le promoteur d'absorption transcutanée ainsi que de s'éliminer rapidement par évaporation au contact de la peau.

16. Composition pharmaceutique selon la Revendication 15, **caractérisée en ce que**:
• le polymère ou copolymère cellulosique est l'éthylcellulose
• le principe actif est l'ibuprofène
• le promoteur d'absorption transcutanée est le 2-éthyl-hexanoate de 2-éthyl-hexyle
• le solvant physiologiquement acceptable est l'isopropanol.

17. Composition selon une des Revendications 1 à 16 **caractérisée en ce qu'**elle est appliquée par pulvérisation directe sans l'aide de gaz propulseur comprimé ou liquéfié.

18. Composition pharmaceutique selon une des Revendications 1 à 17, **caractérisée en ce qu'**elle contient en surplus une fraction aromatisante constituée d'un ou de plusieurs composés aromatisants.

19. Composition pharmaceutique selon la Revendication 18, **caractérisée en ce que** la fraction aromatisante est présente à des concentrations ne dépassant pas 5% du poids de la composition.

20. Composition pharmaceutique selon la Revendication 18 ou 19, **caractérisée en ce que** la fraction aromatisante est constituée par le lévomenthol.

## Patentansprüche

1. Pharmazeutische Zusammensetzung zur transdermalen Verabreichung, **dadurch gekennzeichnet, dass** sie Folgendes umfasst:
• eine polymere Freisetzungsmatrix aus Ethylcellulose, die in der Lage ist, nach dem Trocknen einen weichen Film zu bilden, wobei diese Matrix in einer Menge von 0,5 bis 2 Gew.-% der Zusammensetzung vorliegt;
• Ibuprofen als Wirkstoff;
• einen Promotor der transkutanen Resorption des Wirkstoffs, ausgewählt aus:
(i) einem in dem physiologisch verträglichen nichtwässrigen Lösungsmittel bzw. in den physiologisch verträglichen nichtwässrigen Lösungsmitteln löslichen aliphatischen Fettsäureester, der insgesamt 10 bis 30 Kohlenstoffatome aufweist und gegebenenfalls durch eine oder zwei Hydroxyl-, Carboxyl- oder C₁-C₄-Acyloxygruppen substituiert oder gegebenenfalls durch eine oder zwei ethylenische Bindungen oder ein oder zwei Ether-Sauerstoffatome unterbrochen ist,
(ii) einem in dem physiologisch verträglichen nichtwässrigen Lösungsmittel bzw. in den physiologisch verträglichen nichtwässrigen Lösungsmitteln löslichen aliphatischen C₁₀-C₃₀-Fettalkohol, der gegebenenfalls durch eine oder zwei Hydroxyl-, Carboxyl- oder C₁-C₄-Acyloxygruppen substituiert oder gegebenenfalls durch eine oder zwei ethylenische Bindungen oder ein oder zwei Ether-Sauerstoffatome unterbrochen ist,
• Wasser, das in einer Menge von 3 bis 10 Gew.-% der Zusammensetzung vorliegt,
• zumindest Isopropanol als physiologisch verträgliches nichtwässriges Lösungsmittel, das in der Lage ist, die Freisetzungsmatrix, den Wirkstoff und den Promotor der transkutanen Resorption zu lösen und sich beim Kontakt mit der Haut schnell durch Verdampfen zu verflüchtigen.

2. Pharmazeutische Zusammensetzung nach Anspruch 1, **dadurch gekennzeichnet, dass** die polymere Freisetzungsmatrix in einer Menge von 0,5 bis 1 Gew.-% der Zusammensetzung vorliegt.

3. Pharmazeutische Zusammensetzung nach einem der Ansprüche 1 oder 2, **dadurch gekennzeichnet, dass** der Wirkstoff in einer Konzentration von höchstens 15 Gew.-% der Zusammensetzung vorliegt.

4. Pharmazeutische Zusammensetzung nach Anspruch 3, **dadurch gekennzeichnet, dass** der Wirkstoff in einer Menge von 3 bis 10 Gew.-% der Zusammensetzung vorliegt.

5. Pharmazeutische Zusammensetzung nach einem der Ansprüche 1 bis 4, **dadurch gekennzeichnet, dass** der Promotor der transkutanen Resorption in einer Konzentration von höchstens 40 Gew.-% der Zusammensetzung vorliegt.

6. Pharmazeutische Zusammensetzung nach Anspruch 5, **dadurch gekennzeichnet, dass** der Promotor der transkutanen Resorption in einer Menge von 10 bis 30 Gew.-% der Zusammensetzung vorliegt.

7. Pharmazeutische Zusammensetzung nach Anspruch 6, **dadurch gekennzeichnet, dass** der Promotor der transkutanen Resorption in einer Menge von 15 bis 25 Gew.-% der Zusammensetzung vorliegt.

8. Pharmazeutische Zusammensetzung nach einem der Ansprüche 1 bis 7, **dadurch gekennzeichnet, dass** das physiologisch verträgliche nichtwässrige Lösungsmittel in einer zur Erreichung von 100 Gew.-% der Zusammensetzung ausreichenden Menge vorliegt.

9. Pharmazeutische Zusammensetzung nach einem der Ansprüche 1 bis 8, **dadurch gekennzeichnet, dass** das Ibuprofen in einer Menge von 4 bis 10 Gew.-% der Zusammensetzung vorliegt.

10. Pharmazeutische Zusammensetzung nach Anspruch 9, **dadurch gekennzeichnet, dass** das Ibuprofen in einer Menge von 4 bis 5 Gew.-% der Zusammensetzung vorliegt.

11. Pharmazeutische Zusammensetzung nach Anspruch 10, **dadurch gekennzeichnet, dass** der Promotor der transkutanen Resorption ausgewählt ist aus:
• einem in dem physiologisch verträglichen nichtwässrigen Lösungsmittel bzw. in den physiologisch verträglichen nichtwässrigen Lösungsmitteln löslichen aliphatischen Fettsäureester der allgemeinen Formel: 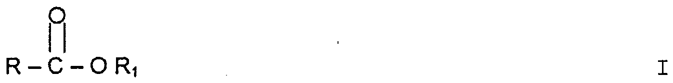 in der R für eine geradkettige oder verzweigte C₂-C₁₇-Alkyl- oder C₂-C₁₇-Alkenylgruppe, die gegebenenfalls durch eine Hydroxyl-, Carboxyl-oder C₁-C₄-Acyloxygruppe substituiert ist, steht und R₁ für eine geradkettige oder verzweigte C₃-C₈-Alkylgruppe, die gegebenenfalls durch eine oder zwei Hydroxylgruppen substituiert ist, steht oder R₁ für eine Gruppe -CH₂-CH₂-O-(CH₂)₂-O-CH₂-CH₃ steht, wobei der aliphatische Fettsäureester mindestens 10 Kohlenstoffatome und maximal 2 Hydroxylgruppen aufweist,
• einem in dem physiologisch verträglichen nichtwässrigen Lösungsmittel bzw. in den physiologisch verträglichen nichtwässrigen Lösungsmitteln löslichen aliphatischen Fettalkohol der allgemeinen Formel:
R₂-OH II
in der R₂ für eine C₁₀-C₂₀-Alkylgruppe steht.

12. Pharmazeutische Zusammensetzung nach Anspruch 11, **dadurch gekennzeichnet, dass** R₁ für eine Isopropyl-, 2-Ethylhexyl- oder 1,2-Dihydroxyethylgruppe steht.

13. Pharmazeutische Zusammensetzung nach einem der Ansprüche 9 oder 10, **dadurch gekennzeichnet, dass** der Promotor der transkutanen Resorption ausgewählt ist aus:
• 2-Ethylhexansäure-2-ethylhexylester
• Isopropylmyristat
• Diethylenglykolmonoethylethermyristat
• Isopropylpalmitat
• 2-Octyldodecanol
• Undecylensäure-2-ethylhexylester
• Bernsteinsäure-2-ethylhexylester
• 12-Hydroxystearinsäure-2-ethylhexylester
• 12-Acetoxystearinsäure-2-ethylhexylester
• Glycerinisostearat
• Hexyllaurat.

14. Pharmazeutische Zusammensetzung nach Anspruch 10, 11 oder 12, **dadurch gekennzeichnet, dass** es sich bei dem Promotor der transkutanen Resorption um 2-Ethylhexansäure-2-ethylhexylester handelt.

15. Pharmazeutische Zusammensetzung zur transdermalen Verabreichung, **dadurch gekennzeichnet, dass** sie Folgendes umfasst:
a) 0,5 bis 2 Gew.-% einer polymeren Freisetzungsmatrix aus Ethylcellulose, die in der Lage ist, nach dem Trocknen einen weichen Film zu bilden,
b) 3 bis 10 Gew.-% Ibuprofen als Wirkstoff,
c) 10 bis 30 Gew.-% eines Promotors der transkutanen Resorption des Wirkstoffs, ausgewählt aus:
• 2-Ethylhexansäure-2-ethylhexylester
• Isopropylmyristat
• Diethylenglykolmonoethylethermyristat
• Isopropylpalmitat
• 2-Octyldodecanol
• Undecylensäure-2-ethylhexylester
• Bernsteinsäure-2-ethylhexylester
• 12-Hydroxystearinsäure-2-ethylhexylester
• Hexyllaurat
• Glycerinisostearat
• 12-Acetoxystearinsäure-2-ethylhexylester,
d) 3 bis 10 Gew.-% Wasser,
e) eine zur Erreichung von 100 Gew.-% der Zusammensetzung ausreichende Menge von zumindest Isopropanol als physiologisch verträglichem nichtwässrigem Lösungsmittel, das in der Lage ist, die Freisetzungsmatrix, den Wirkstoff und den Promotor der transkutanen Resorption zu lösen und sich beim Kontakt mit der Haut schnell durch Verdampfen zu verflüchtigen.

16. Pharmazeutische Zusammensetzung nach Anspruch 15, **dadurch gekennzeichnet, dass**:
• es sich bei dem Cellulosepolymer oder -copolymer um Ethylcellulose handelt,
• es sich bei dem Wirkstoff um Ibuprofen handelt,
• es sich bei dem Promotor der transkutanen Resorption um 2-Ethylhexansäure-2-ethylhexyl-ester handelt,
• es sich bei dem physiologisch verträglichen Lösungsmittel um Isopropanol handelt.

17. Zusammensetzung nach einem der Ansprüche 1 bis 16, **dadurch gekennzeichnet, dass** sie durch direktes Aufsprühen ohne Hilfe eines verdichteten oder verflüssigten Treibgases aufgebracht wird.

18. Pharmazeutische Zusammensetzung nach einem der Ansprüche 1 bis 17, **dadurch gekennzeichnet, dass** sie außerdem eine aromatisierende Fraktion, die aus einer oder mehreren aromatisierenden Verbindungen besteht, enthält.

19. Pharmazeutische Zusammensetzung nach Anspruch 18, **dadurch gekennzeichnet, dass** die aromatisierende Fraktion in Konzentrationen von höchstens 5 Gew.-% der Zusammensetzung vorliegt.

20. Pharmazeutische Zusammensetzung nach Anspruch 18 oder 19, **dadurch gekennzeichnet, dass** die aromatisierende Fraktion aus Levomenthol besteht.

## Claims

1. Pharmaceutical composition for transdermal administration, **characterized in that** it comprises:
• an ethylcellulose polymeric release matrix capable of forming a supple film after drying, this matrix being present in a proportion of from 0.5% to 2% of the weight of the composition
• ibuprofen as active principle
• a promoter of transcutaneous absorption of the active principle, chosen from:
(i) an aliphatic fatty acid ester, which is soluble in the physiologically acceptable non-aqueous solvent(s), containing in total from 10 to 30 carbon atoms and being optionally substituted with one or two hydroxyl, carboxylic or C₁-C₄ acyloxy groups or optionally interrupted with one or two ethylenic bonds or with one or two ether oxygens,
(ii) a C₁₀-C₃₀ aliphatic fatty alcohol, which is soluble in the physiologically acceptable non-aqueous solvent(s), and optionally substituted with one or two hydroxyl, carboxylic or C₁-C₄ acyloxy groups or optionally interrupted with one or two ethylenic bonds or with one or two ether oxygens.
• water present in a proportion of from 3% to 10% of the weight of the composition
• at least isopropanol as a physiologically acceptable non-aqueous solvent capable of dissolving the release matrix, the active principle and the transcutaneous absorption promoter, and also of being rapidly eliminated by evaporation on contact with the skin.

2. Pharmaceutical composition according to Claim 1, **characterized in that** the polymer release matrix is present in a proportion of from 0.05% to 1% of the weight of the composition.

3. Pharmaceutical composition according to either of Claims 1 and 2, **characterized in that** the active principle is present at a concentration not exceeding 15% of the weight of the composition.

4. Pharmaceutical composition according to Claim 3, **characterized in that** the active principle is present in a proportion of from 3% to 10% of the weight of the composition.

5. Pharmaceutical composition according to one of Claims 1 to 4, **characterized in that** the transcutaneous absorption promoter is present at a concentration not exceeding 40% of the weight of the composition.

6. Pharmaceutical composition according to Claim 5, **characterized in that** the transcutaneous absorption promoter is present in a proportion of from 10% to 30% of the weight of the composition.

7. Pharmaceutical composition according to Claim 6, **characterized in that** the transcutaneous absorption promoter is present in a proportion of from 15% to 25% of the weight of the composition.

8. Pharmaceutical composition according to one of Claims 1 to 7, **characterized in that** the physiologically acceptable non-aqueous solvent is present in an amount that is sufficient to reach 100% of the weight of the composition.

9. Pharmaceutical composition according to one of Claims 1 to 8, **characterized in that** the ibuprofen is present in a proportion of from 4% to 10% of the weight of the composition.

10. Pharmaceutical composition according to Claim 9, **characterized in that** the ibuprofen is present in a proportion of from 4% to 5% of the weight of the composition.

11. Pharmaceutical composition according to Claim 10, **characterized in that** the transcutaneous absorption promoter is chosen from:
• an aliphatic fatty acid ester that is soluble in the physiologically acceptable non-aqueous solvent(s) and of general formula: 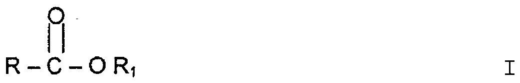 in which R represents a linear or branched C₂-C₁₇ alkyl or alkenyl group optionally substituted with a hydroxyl, carboxylic or C₁-C₄ acyloxy group and R₁ represents a linear or branched C₃-C₈ alkyl group optionally substituted with one or two hydroxyl groups or R₁ represents a
- CH₂-CH₂-O-(CH₂)₂-O-CH₂-CH₃ group, the aliphatic fatty acid ester containing a minimum of 10 carbon atoms and a maximum of 2 hydroxyl groups,
• an aliphatic fatty alcohol, which is soluble in the physiologically acceptable non-aqueous solvent(s) and of general formula:
R₂-OH II
in which R₂ represents a C₁₀-C₂₀ alkyl group.

12. Pharmaceutical composition according to Claim 11, **characterized in that** R₁ represents an isopropyl, 2-ethylhexyl or 1,2-dihydroxyethyl group.

13. Pharmaceutical composition according to either of Claims 9 and 10, **characterized in that** the transcutaneous absorption promoter is chosen from:
• 2-ethylhexyl 2-ethylhexanoate
• isopropyl myristate
• diethylene glycol monoethyl ether myristate
• isopropyl palmitate
• 2-octyldodecanol
• 2-ethylhexyl undecylenate
• 2-ethylhexyl succinate
• 2-ethylhexyl 12-hydroxystearate
• 2-ethylhexyl 12-acetoxystearate
• glyceryl isostearate
• hexyl laurate.

14. Pharmaceutical composition according to Claim 10, 11 or 12, **characterized in that** the transcutaneous absorption promoter is 2-ethylhexyl 2-ethylhexanoate.

15. Pharmaceutical composition for transdermal administration, **characterized in that** it comprises, by weight:
a) from 0.5% to 2% of an ethylcellulose polymeric release matrix capable of forming a supple film after drying, chosen from cellulose-based polymers or copolymers
b) from 3% to 10% of ibuprofen as active principle
c) from 10% to 30% of a promoter of transcutaneous absorption of the active principle chosen from:
• 2-ethylhexyl 2-ethylhexanoate
• isopropyl myristate
• diethylene glycol monoethyl ether myristate
• isopropyl palmitate
• 2-octyldodecanol
• 2-ethylhexyl undecylenate
• 2-ethylhexyl succinate
• 2-ethylhexyl 12-hydroxystearate
• hexyl laurate
• glyceryl isostearate
• 2-ethylhexyl 12-acetoxystearate
d) from 3% to 10% of water
e) a sufficient amount, to reach 100% of the weight of the composition, of at least isopropanol as a physiologically acceptable non-aqueous solvent capable of dissolving the release matrix, the active principle and the transcutaneous absorption promoter, and also of being rapidly eliminated by evaporation on contact with the skin.

16. Pharmaceutical composition according to Claim 15, **characterized in that**:
• the cellulose-based polymer or copolymer is ethylcellulose
• the active principle is ibuprofen
• the transcutaneous absorption promoter is 2-ethylhexyl 2-ethylhexanoate
• the physiologically acceptable solvent is isopropanol.

17. Composition according to one of Claims 1 to 16, **characterized in that** it is applied by direct spraying, without the aid of a compressed or liquefied propellant gas.

18. Pharmaceutical composition according to one of Claims 1 to 17, **characterized in that** it also contains an aromatizing fraction consisting of one or more aromatizing compounds.

19. Pharmaceutical composition according to Claim 18, **characterized in that** the aromatizing fraction is present at concentrations not exceeding 5% of the weight of the composition.

20. Pharmaceutical composition according to Claim 18 or 19, **characterized in that** the aromatizing fraction consists of levomenthol.
